# EUROPÄISCHE PATENTSCHRIFT

(11) **EP 3 753 660 B1**
(45) Veröffentlichungstag und Bekanntmachung des Hinweises auf die Patenterteilung: **26.02.2025**
(21) Anmeldenummer: 19181566.1
(22) Anmeldetag: 20.06.2019
(51) Int. Cl.: B23D 61/00, A61B 17/14, B23D 61/02

(54) **SCHNEIDWERKZEUG**
CUTTING TOOL
OUTIL DE COUPE

(43) Veröffentlichungstag der Anmeldung: 23.12.2020
(73) Patentinhaber: CERATIZIT Luxembourg S.à r.l., 8232 Mamer (LU)
(72) Erfinder: DRÖSCHEL, Michael, 8232 Mamer (LU); MAGIN, Michael, 8232 Mamer (LU); SOGAARD, Michael, 8232 Mamer (LU); SCHELLER, Luc, 8232 Mamer (LU)
(74) Vertreter: Ciesla, Dirk

(56) Entgegenhaltungen:
- EP-A2- 1 558 419
- DE-A1- 102013 203 613
- US-A- 5 178 626
- US-A1- 2010 010 492
- US-A1- 2019 054 552

## Beschreibung

Die vorliegende Erfindung betrifft ein Schneidwerkzeug zum Trennen eines nichtmetallischen Materials, umfassend ein Halterungselement, ein an dem Halterungselement zur schneidenden Kontaktierung des nichtmetallischen Materials gehaltertes Schneidelement und einen an einen Halterungsmaterialbereich des Halterungselements sowie einen Schneidmaterialbereich des Schneidelements angrenzenden Grenzbereich.

Die vorliegende Erfindung liegt auf dem Gebiet des Trennens, insbesondere indem ein Schneidwerkzeug der eingangs genannten Art in eine oszillierende Schwingung einer Frequenz von beispielsweise 20000 Hüben pro Minute oder mehr bei relativ kleiner Amplitude versetzt wird, eines nichtmetallischen Materials, insbesondere eines solchen, welches üblicherweise mit einer relativ geringen Schnittkraft, insbesondere zerspanend, getrennt werden kann, wie beispielsweise ein Holz oder Hölzer, ein Kunststoff oder Kunststoffe, ein Faserverbundwerkstoff oder Faserverbundwerkstoffe, ein organisches Material oder organische Materialien, ein Gips oder Gipse, ein Gasbeton oder Gasbetons, also ein poröser Beton bzw. Betons, jeweils auf Basis von Kalk-, Kalkzement- oder Zementmörtel, oder ein Knochenmaterial oder Knochenmaterialien jeweils menschlichen oder tierischen Ursprungs.

Zu den Schneidwerkzeugen der eingangs genannten Art gehören danach insbesondere entsprechende Sägeblätter oder Klingen.

Aus der EP 2 295 211 B1 ist ein Schneidwerkzeug der eingangs genannten Art in Form eines Sägeblatts vorbekannt. Das Trennen eines nichtmetallischen Materials, insbesondere - aber nicht nur - wenn dieses ein Knochenmaterial menschlichen oder tierischen Ursprungs ist, mit einem solchen Schneidwerkzeug wird aber dadurch erschwert, dass dieses mitunter in unkontrollierter Weise von einer üblicherweise - aber nicht nur - mittels manueller Führung vorgegebenen Trenn- oder Schnittrichtung abweicht. Ein solches Abweichen kann auch als Ausbrechen des Schneidwerkzeugs bezeichnet werden.

Die EP 1 558 419 A2 zeigt ein Knochensägeblatt.

Die US 2019/054552 A1 zeigt eine oszillierende Klinge.

Die DE 10 2013 203613 A1 zeigt ein Schneidwerkzeug nach dem Oberbegriff des Anspruchs 1, insbesondere ein Tauchsägeblatt.

Die US 5 178 626 A zeigt ein chirurgisches Sägeblatt.

Die US 2010/010492 A1 zeigt einen Miniaturhäcksler für medizinische Anwendungen.

Die Aufgabe der vorliegenden Erfindung ist es daher, ein Schneidwerkzeug der eingangs genannten Art bereitzustellen, mit welchem ein präziseres Trennen, insbesondere - aber nicht nur - von Knochenmaterialien menschlichen oder tierischen Ursprungs, möglich ist.

Diese Aufgabe wird durch ein Schneidwerkzeug mit den Merkmalen nach Anspruch 1 gelöst. Vorteilhafte Weiterbildungen sind den abhängigen Ansprüchen zu entnehmen.

Das Schneidwerkzeug zum Trennen eines nichtmetallischen Materials umfasst ein Halterungselement, ein an dem Halterungselement zur schneidenden Kontaktierung des nichtmetallischen Materials gehaltertes Schneidelement und einen an einen Halterungsmaterialbereich des Halterungselements sowie einen Schneidmaterialbereich des Schneidelements angrenzenden Grenzbereich, wobei ein Verformungswiderstand gegen eine elastische Verformung des Halterungsmaterialbereichs größer ist als ein Verformungswiderstand gegen eine elastische Verformung des Schneidmaterialbereichs. Dadurch wird das Schneidwerkzeug im Bereich des Halterungselements, welches zum Beispiel plattenförmig ausgeformt sein kann, gegenüber einer elastischen Verformung stabilisiert, so dass das Schneidelement, welches Schneidzähne aufweisen kann, gegen das starrere Halterungselement im Sinne eines Widerlagers beim Trennen gedrückt wird, was zum Beispiel ein Trennen entlang einer vorgegebenen, insbesondere geraden, Trennlinie begünstigt; diese Trennlinie kann insbesondere auf und/oder an einem Knochen, einem Holzwerkstück, einem Kunststoffwerkstück, einem Faserverbundwerkstoffwerkstück, einem organischen Werkstück, einem Gipswerkstück oder einem Gasbetonwerkstück markiert sein. Dies kann noch weiter verbessert werden, indem das Schneidelement entlang einer Längsrichtung des Schneidwerkzeugs kürzer bemessen ist als das Halterungselement, insbesondere wenn das Schneidelement und das Halterungselement jeweils plattenförmig ausgestaltet sind. Das Halterungselement und das Schneidelement sind stoffschlüssig miteinander verbunden. Der Grenzbereich umfasst oder besteht aus einem Stoffschlussmaterial, wie einem Lot und/oder einem Klebematerial, oder einer Materialmischung, umfassend oder bestehend aus Materialien des Halterungselements und des Schneidelements sowie optionalen weiteren Materialien, welche aus einer Stoffschlussverbindungsquelle, zum Beispiel einem Schweißmittel, insbesondere einem Schweißdraht, erhältlich sind. Letzteres ist zum Beispiel durch Herstellung einer Schweißverbindung zwischen dem Halterungselement und dem Schneidelement erhältlich, wonach in diesem Beispiel der Grenzbereich als Schweißnaht oder dergleichen bezeichnet werden kann.

Vorzugsweise sind der Halterungsmaterialbereich und der Schneidmaterialbereich gegenüberliegend voneinander angeordnet, insbesondere in Draufsicht auf das Schneidwerkzeug. Dadurch wird das Schneidelement noch stabiler gehaltert.

Mit dem Grenzbereich, welcher auch als erster Grenzbereich bezeichnet werden kann, ist insbesondere ein Bereich gemeint, welcher zumindest abschnittsweise aufgrund einer kraftschlüssigen und/oder formschlüssigen und/oder stoffschlüssigen Verbindung zwischen dem Halterungselement und dem Schneidelement gebildet ist oder werden kann. In Draufsicht auf das Schneidwerkzeug, insbesondere wenn das Halterungselement plattenförmigen ausgebildet ist, kann einem Verlauf des Grenzbereichs insbesondere eine Verbindungslinie, welche innerhalb des Grenzbereichs verläuft, zugeordnet werden, wobei die Verbindungslinie vorzugsweise mindestens zwei, vorzugsweise diametrale Außenkanten, des Schneidwerkzeugs miteinander verbindet. Vorzugsweise mündet die Verbindungslinie in mindestens einen Außenkantenbereich des Schneidwerkzeugs. Die Verbindungslinie kann gerade, gekrümmt oder abschnittsweise gerade oder abschnittsweise gekrümmt verlaufend sein. Die Verbindungslinie kann quer oder parallel zur Längsachse verlaufen. Der Verbindungslinie kann auch eine zwischen diesen beiden Orientierungen liegende Orientierung zugeordnet werden.

Der Grenzbereich kann einen Materialmischungsbereich umfassen oder vorzugsweise aus diesem bestehen. Der Materialmischungsbereich kann vorzugsweise auf Material des Halterungsmaterialbereichs und Material des Schneidelementbereichs basieren, vorzugsweise daraus bestehen. Alternativ oder ergänzend kann der Materialmischungsbereich auf Material eines Stoffschlussmaterials, beispielsweise eines Lots und/oder eines Klebematerials, basieren, vorzugsweise daraus bestehen.

Besonders bevorzugt ist das Schneidwerkzeug als Oszillationssägeblatt ausgeformt, wobei das Oszillationssägeblatt über eine Antriebswelle, insbesondere eine Motorwelle, einer Antriebseinheit, insbesondere eines Motor, vorzugsweise eines Elektromotors, in oszillierende Schwingung mit einem vorgegebenen Hub versetzbar ist, so dass das Schneidelement im Bereich eines freien Endes, welches Schneidzähne, welche auch als Sägezähne bezeichnet werden können, aufweist, parallel und/oder senkrecht zu einer Trennrichtung, welche auch als Schnittrichtung bezeichnet werden kann, schwingbar ist. Denn auf diese Weise wird ein besonders präzises oszillierendes Trennen des nichtmetallischen Materials ermöglicht.

Noch bevorzugter ist es danach, wenn das als Oszillationssägeblatt ausgeformte Schneidwerkzeug mit dem Motor wirkmäßig verbunden ist, so dass auf diese Weise eine Oszillationssäge bereitgestellt wird. Besonders bevorzugt ist es mit dem Motor über ein Getriebe, insbesondere eine Getriebewelle des Getriebes, wirkmäßig verbunden.

Mit der schneidenden Kontaktierung ist insbesondere gemeint, dass das nichtmetallische Material zerspant wird.

Indem das Schneidwerkzeug das Halterungselement und das Schneidelement umfasst, ist es dementsprechend zweiteilig ausgebildet. Denkbar und auch möglich ist aber auch eine mehrteilige Ausbildung mit drei oder mehr Elementen, wie beispielsweise einem zusätzlichen Verbindungselement zum formschlüssigen Verbinden mit einem Antriebselement einer Antriebseinheit. Gemäß einer Weiterbildung des Schneidwerkzeugs ist der Verformungswiderstand des Halterungsmaterialbereichs und der Verformungswiderstand des Schneidmaterialbereichs jeweils ein Biegeverformungswiderstand. Danach wird das Trennen des nichtmetallischen Materials insbesondere gegen eine Biegeverformung stabilisiert. Eine solche Stabilisierung kann insbesondere dadurch bereitgestellt werden, dass das Produkt aus einem Elastizitätsmodul eines Materials des Halterungsmaterialbereichs und einem Flächenträgheitsmoment des Halterungsmaterialbereichs größer ist als das Produkt aus einem Elastizitätsmodul eines Materials des Schneidmaterialbereichs und einem Flächenträgheitsmoment des Schneidmaterialbereichs. Diese Produkte können auch als Biegesteifigkeiten des Halterungsmaterialbereichs bzw. des Schneidmaterialbereichs bezeichnet werden. Die Flächenträgheitsmomente sind dabei jeweils bezüglich einer Biegeachse, welche im geometrischen Sinne definiert ist, vorzugsweise bestimmt, die bis auf ihre Position entlang einer Längsachse des Schneidwerkzeugs bezüglich des Halterungselements und des Schneidelements jeweils in derselben Weise positioniert ist, zum Beispiel auf der Hälfte einer im Querschnittschnitt des Halterungselements und des Schneidelements bemessenen Höhe im Falle einer horizontalen Biegeachse.

Der Halterungsmaterialbereich besteht aus einem Halterungsmaterial und der Schneidmaterialbereich besteht aus einem dem Halterungsmaterial verschiedenen Schneidmaterial. Dadurch kann einerseits die Biegesteifigkeit des Schneidwerkzeugs erhöht werden, indem das Halterungsmaterial beispielsweise auf Basis seines Elastizitätsmoduls ausgewählt wird, und andererseits das Schneidmaterial auf Basis seiner Eignung, das für das schneidende Kontaktieren des nichtmetallischen Materials vorgesehene Schneidelement zu optimieren, ausgewählt werden. Letztere Eignung kann beispielsweise dadurch ausgedrückt werden, dass Schneidzähne mit definierten Schneidzahnflanken, insbesondere in reprozierbarer Weise, hergestellt werden können. Hierfür kommt insbesondere Stahl als Schneidmaterial in Betracht.

Vorzugsweise besteht das Halterungselement aus dem Halterungsmaterial, was zu einer noch besseren Stabilisierung des Schneidelements beim Trennen beiträgt.

Vorzugsweise besteht das Schneidelement aus dem Schneidmaterial, wonach das Schneidelement bei geeigneter Wahl des Schneidmaterials für das Trennen eines bestimmten nichtmetallischen Materials und/oder zur Aufnahme von Reaktionskräften und thermischen Belastungen, welche auf das Schneidelement beim Trennen wirken können, ausgewählt werden kann.

Ein Elastizitätsmodul des Halterungsmaterials ist größer als ein Elastizitätsmodul des Schneidmaterials. Dadurch kann in vorteilhafter Weise die Biegesteifigkeit des Schneidwerkzeugs unter vorteilhafter Vermeidung besonderer konstruktiver Maßnahmen erhöht werden. So kann der Elastizitätsmodul des Halterungsmaterials beispielsweise um einen Faktor 1,1 bis 5 oder mehr größer sein als der Elastizitätsmodul des Schneidmaterials. Bevorzugte Werte dieses Bereichs, also des Faktors, sind 1,1, 1,2, 1,3, 1,4, 1,5, 1,6, 1,7, 1,8, 1,9, 2,0, 2,1, 2,2, 2,3, 2,4, 2,5, 2,6, 2,7, 2,8, 2,9, 3,0, 3,1, 3,2, 3,3, 3,4, 3,5, 3,6, 3,7, 3,8, 3,9, 4,0, 4,1, 4,2, 4,3, 4,4, 4,5, 4,6, 4,7, 4,8, 4,9 oder 5,0.

Mit dem Elastizitätsmodul des Schneidmaterials und des Halterungsmaterials sind insbesondere auch die für die jeweilige elastische Verformung des Schneidmaterialbereichs und des Halterungsmaterialbereichs relevante Komponente oder Komponenten eines dem Schneidmaterial und dem Halterungsmaterial jeweils zuordenbaren Elastizitätstensors gemeint, sofern das Schneidmaterial bzw. das Halterungsmaterial elastisch anisotrop sind.

Vorzugsweise besteht das Halterungselement aus dem Halterungsmaterialbereich, was zu einer noch besseren Stabilisierung des Schneidwerkzeugs beim Trennen beiträgt.

Vorzugsweise besteht das Schneidelement aus dem Schneidmaterialbereich, wonach das Schneidelement bei geeigneter Wahl des Schneidmaterialbereichs für das Trennen eines bestimmten nichtmetallischen Materials und/oder zur Aufnahme entsprechender Reaktionskräfte, welche auf das Schneidelement beim Trennen wirken können, ausgewählt werden kann.

Gemäß einer Weiterbildung des Schneidwerkzeugs weist das Halterungsmaterial eine Materialmatrix mit einer Matrixduktilität und in der Materialmatrix angeordnete Hartstoffpartikel mit einer Partikelduktilität, welche kleiner ist als die Matrixduktilität, auf. Dadurch wird das Halterungsmaterial zur weiteren Erhöhung der Biegesteifigkeit und Steigerung der Verschleißfestigkeit des Schneidwerkzeugs in Form eines Verbundwerkwerkstoffs bereitgestellt. Die Hartstoffpartikel können zum Beispiel aus Wolframcarbid (WC) und die Materialmatrix aus Cobalt (Co) oder einem Mischkristall auf Basis von Cobalt (Co) bestehen. Danach handelt es sich bei dem Halterungsmaterial um ein Hartmetall (*cemented carbide*)*.* Vorzugsweise sind die Hartstoffpartikel aus WC kleiner oder gleich 20 µm, vorzugsweise kleiner oder gleich 20 µm, 19 µm, 18 µm, 17 µm, 16 µm, 15 µm, 14 µm, 13 µm, 12 µm, 11 µm, 10 µm, 9 µm, 8 µm, 7 µm, 6 µm, 5 µm, 4 µm, 3 µm, 2 µm, 1 µm, 0,5 µm, 0,4 µm, 0,3 µm, 0,2 µm oder 0,1 µm. Vorzugsweise beträgt der Cobalt-Gehalt, in elementarer Form oder als Mischkristall, bezogen auf die Gesamtzusammensetzung des Halterungsmaterials 3 bis 30 Gewichtsprozent (bevorzugte Werte, also Gewichtsprozente, dieses Bereichs sind 3, 4, 5, 6, 7, 8, 9, 10, 11, 12, 13, 14, 15, 16, 17, 18, 19, 20, 21, 22, 23, 24, 25, 26, 27, 28, 29 oder 30); den entsprechenden Rest bilden die Hartstoffpartikel aus WC und optional andere Hartstoffpartikel wie Vanadiumcarbid (VC), Chromcarbid (Cr₂C₃) und/oder Tantal-Niob-Carbid (Ta,Nb)C. Alternativ oder ergänzend können die Hartstoffpartikel aus Titancarbid (TiC), Titannitrid (TiN) und/oder Tantalcarbid (TaC) und die Materialmatrix aus Niob (Nb), Molybdän (Mo) oder Kobalt (Co) bzw. entsprechenden Mischkristallen dieser Elemente bestehen. Danach handelt es sich bei dem Halterungsmaterial um ein Cermet (*cermet*)*.* Vorzugsweise sind die Hartstoffpartikel aus Titancarbid (TiC), Titannitrid (TiN) und/oder Tantalcarbid (TaC) kleiner oder gleich 20 µm, vorzugsweise kleiner oder gleich 20 µm, 19 µm, 18 µm, 17 µm, 16 µm, 15 µm, 14 µm, 13 µm, 12 µm, 11 µm, 10 µm, 9 µm, 8 µm, 7 µm, 6 µm, 5 µm, 4 µm, 3 µm, 2 µm, 1 µm, 0,5 µm, 0,4 µm, 0,3 µm, 0,2 µm oder 0,1 µm. Vorzugsweise beträgt der Niob- (Nb), Molybdän- (Mo) oder Kobalt-Gehalt (Co), jeweils in elementarer Form oder als Mischkristall, bezogen auf die Gesamtzusammensetzung des Halterungsmaterials 3 bis 30 Gewichtsprozent (bevorzugte Werte, also Gewichtsprozente, dieses Bereichs sind 3, 4, 5, 6, 7, 8, 9, 10, 11, 12, 13, 14, 15, 16, 17, 18, 19, 20, 21, 22, 23, 24, 25, 26, 27, 28, 29 oder 30); den entsprechenden Rest bilden die Hartstoffpartikel aus Titancarbid (TiC), Titannitrid (TiN) und/oder Tantalcarbid (TaC) und optional andere Hartstoffpartikel. Vorzugsweise ist das Halterungsmaterial im Falle der Bereitstellung als Cermet nickelfrei.

Gemäß einer Weiterbildung des Schneidwerkzeugs ist das Halterungsmaterial ein keramisches Material, ein Refraktärmetall oder eine Mischung daraus.

Dadurch wird das Halterungsmaterial zur weiteren Erhöhung der Biegesteifigkeit bereitgestellt. Bei dem Refraktärmetall kann es sich zum Beispiel um ein Molybdän- oder Wolfram-Metall handeln, welches sich jeweils in vorteilhafterweise zu dem Halterungselement um- und/oder urformen lässt und zugleich einen relativ hohen Elastizitätsmodul zur vorteilhaften Steigerung der Biegesteifigkeit aufweist. Bei der Keramik kann es sich zum Beispiel um eine Oxidkeramik auf Basis von Aluminiumoxid, Magnesiumoxid, Zirkoniumoxid oder Titandioxid handeln. Vorzugsweise ist die Oxidkeramik eine mit Zirkonium verstärkte Aluminiumoxid-Keramik. Alternativ kann es sich bei der Keramik um eine im Wesentlichen, vorzugsweise gänzlich, oxidfreie Keramik handeln, also insbesondere eine Keramik auf Basis von Carbiden, Nitriden, Boriden und/oder Siliciden.

Gemäß einer Weiterbildung des Schneidwerkzeugs ist das Halterungselement monolithisch. Danach wird ein besonders biegesteifes Schneidwerkzeug bereitgestellt. Mit monolithisch ist insbesondere gemeint, dass von einem beliebigen Ende des Halterungselements beginnend nach einem anderen beliebigen Ende des Halterungselements ein homogener Materialpfad gebildet ist.

Gemäß einer Weiterbildung des Schneidwerkzeugs ist das Schneidmaterial ein metallisches Material, insbesondere ein Stahl, und ist eine Duktilität des Schneidmaterials größer als eine Duktilität des Halterungsmaterials. Dadurch wird das Schneidmaterial gegenüber dem Halterungsmaterial als relativ duktiles Material bereitgestellt, wonach zum Beispiel die Herstellung von Schneidzähnen in und/oder an einem Schneidelementrohkörper, aus welchem das Schneidelement herstellbar ist oder hergestellt ist, mit definierten Zahnflanken in erleichterter, vorzugsweise reproduzierbarer, Weise ermöglicht wird, insbesondere durch Stanzen in Kombination mit optionalen weiteren Herstellungsschritten wie Schleifen, Fräsen, Sägen oder dergleichen. Die Duktilität des Schneidmaterials und des Halterungsmaterials können dabei anhand einer aus einem Zugversuch erhältlichen Gleichmaßdehnung oder Bruchdehnung bemessen sein. Die Bruchdehnung des Schneidmaterials in Form des metallischen Materials, insbesondere des Stahls, ist vorzugsweise um mindestens einen Faktor 2 bis 50 größer als die Bruchdehnung des Halterungsmaterials. Bevorzugte Werte dieses Bereichs, also des Faktors, sind 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3 oder 2.

Gemäß einer Weiterbildung des Schneidwerkzeugs sind der Halterungsmaterialbereich und der Schneidmaterialbereich mittels einer Klebeverbindung, einer Lötverbindung oder einer Schweißverbindung, insbesondere einer Strahlschweißverbindung, stoffschlüssig miteinander verbunden. Dadurch wird jeweils eine besonders vorteilhafte stoffschlüssige Verbindung zwischen dem Halterungsmaterialbereich und dem Schneidmaterialbereich bereitgestellt. Mit der Strahlschweißverbindung ist insbesondere gemeint, dass eine zum Aufschmelzen des Halterungsmaterials und/oder des Schneidmaterials erforderliche Schmelzenergie aus einem oder mehreren Laserstrahlen und/oder Elektronenstrahlen erhältlich ist.

Gemäß einer Weiterbildung des Schneidwerkzeugs ist der Grenzbereich zwischen zwei Außenkantenpunkten des Schneidwerkzeugs erstreckend ausgeformt. Dies ist eine für die, insbesondere stoffschlüssige, stabile Verbindung des Schneidelements mit dem Halterungselement besonders vorteilhafte Maßnahme. Dies zum Beispiel im Sinne einer Schweißnaht oder dergleichen. Vorzugsweise erstrecken sich der Halterungsmaterialbereich und der Schneidelementmaterialbereich zwischen den beiden Außenkantenpunkten.

Gemäß einer Weiterbildung des Schneidwerkzeugs ist der Grenzbereich in einen ersten Außenkantenbereich des Schneidwerkzeugs und in einen zweiten Außenkantenbereich des Schneidwerkzeugs mündend. Dies ist eine für die, insbesondere stoffschlüssige, stabile Verbindung des Schneidelements mit dem Halterungselement besonders vorteilhafte Maßnahme. Die beiden Außenkantenbereiche können an derselben oder verschiedenen Längsachsenpositionen bezüglich einer Längsachse des Schneidwerkzeugs angeordnet sein. Die beiden Außenkantenbereiche können mittels einer Verbindungslinie verbunden werden, welche parallel, senkrecht oder in einem von 90° verschiedenen Winkel, zum Beispiel 0° bis 89°, vorzugsweise 1°, 2°, 3°, 4°, 5°, 10°, 15°, 20°, 25°, 30°, 35°, 40°, 45°, 50°, 55°, 60°, 65°, 70°, 75°, 85°, 89°, gegenüber einer Längsachse des Schneidwerkzeugs orientiert ist. Die beiden Außenkantenbereiche können vorzugsweise jeweils Oberflächenbereiche verschiedener Seitenflächen, welcher vorzugsweise jeweils kleiner sind als eine in einer Draufsicht auf das Halterungselement ersichtliche Oberfläche des Halterungselements, des Schneidwerkzeugs sein, welche in entsprechenden Seitenansichten des Schneidwerkzeugs ersichtlich sind.

Gemäß einer Weiterbildung des Schneidwerkzeugs sind in Draufsicht auf das Schneidwerkzeug mindestens zwei Außenkantenpunkte des Schneidwerkzeugs zwischen dem Halterungsmaterialbereich und dem Schneidmaterialbereich angeordnet. Dadurch bildet der Halterungsmaterialbereich in vorteilhafter Weise zum Beispiel eine Stoffschlussfläche, an welcher das Schneidelement stoffschlüssig mit dem Halterungselement verbunden werden kann oder ist. Die Außenkantenpunkte können mittels einer Verbindungsgeraden verbunden sein, welche senkrecht zur einer Längsachse verläuft oder gegenüber der Längsachse, insbesondere in einer Ebene des Halterungselements, geneigt verläuft, zum Beispiel quer dazu.

Die Stoffschlussfläche, welche vorzugsweise eine Stirnfläche des Halterungselements, welches plattenförmig ausgestaltet sein kann, ist, kann eine dieser korrespondierend ausgeformte Kontaktierungsfläche des Schneidelements im Wesentlichen, vorzugsweise gänzlich, spaltfrei kontaktieren, wonach eine besonders vorteilhafte, weil kontaktierungsmaximierende stoffschlüssige Verbindung zwischen dem Schneidelement und dem Halterungselement realisiert werden kann. Die Stoffschlussfläche und die Kontaktierungsfläche können danach vorzugsweise als jeweils ebene Flächen oder als konvex-konkaves Flächenpaar vorliegen. Im letzteren Falle ist vorzugsweise die Stoffschlussfläche konkav und die Kontaktierungsfläche dementsprechend konvex.

Gemäß einer Weiterbildung des Schneidwerkzeugs weist ein Außenbereich des Halterungsmaterialbereichs zumindest abschnittsweise eine Fase oder eine Kantenverrundung auf. Durch diese Maßnahmen wird in vorteilhafter Weise das Risiko minimiert, dass eine ansonsten rechtwinklige Außenkante des Halterungsmaterialbereichs, zumindest teilweise, bricht und damit weiteres Risswachstum begünstigt.

Gemäß einer Weiterbildung des Schneidwerkzeugs sind zwei Außenkanten des Halterungsmaterialbereichs durch eine konvexe Sehne verbunden. Durch diese Maßnahmen wird in vorteilhafter Weise das Risiko minimiert, dass eine ansonsten rechtwinklige Außenkante des Halterungsmaterialbereichs, zum zumindest teilweise, bricht und damit weiteres Risswachstum begünstigt.

Gemäß einer Weiterbildung des Schneidwerkzeugs umfasst das Schneidwerkzeug ein Verbindungselement zum formschlüssigen Verbinden mit einem Antriebselement einer Antriebeinheit, wobei das Verbindungselement an dem Halterungselement gehaltert ist, ein Grenzbereich, welcher auch als zweiter Grenzbereich zur Unterscheidung von dem an den Halterungsmaterialbereich und den Schneidmaterialbereich angrenzenden Grenzbereich bezeichnet werden kann, zwischen dem Verbindungselement und dem Halterungselement gebildet ist und eine Duktilität eines Verbindungsmaterials des Verbindungselements größer ist als eine Duktilität des Halterungsmaterials. Dadurch wird das Schneidwerkzeug mit Verbinden mit dem Antriebselement, welches insbesondere eine Motorwelle eines Elektromotors oder eine Abtriebswelle eines mit der Motorwelle verbundenen Getriebes sein kann, in vorteilhafterweise bereitgestellt. Ferner wird das Verbindungsmaterial gegenüber dem Halterungsmaterial als relativ duktiles Material bereitgestellt, wonach zum Beispiel die Herstellung einer Ausnehmung zum formschlüssigen Verbinden mit dem Antriebselement besonders einfach, zum Beispiel durch Stanzen, Sägen oder Fräsen, möglich ist. Die Duktilität des Verbindungsmaterials und des Halterungsmaterials können dabei anhand einer aus einem Zugversuch erhältlichen Gleichmaßdehnung oder Bruchdehnung bemessen sein. Die Bruchdehnung des Verbindungsmaterials ist vorzugsweise um mindestens einen Faktor 2 bis 50 größer als die Bruchdehnung des Halterungsmaterials. Bevorzugte Werte dieses Bereichs, also des Faktors, sind 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3 oder 2. Das Verbindungselement ist vorzugsweise plattenförmig. Das Verbindungselement und das Halterungselement sind vorzugsweise formschlüssig miteinander verbunden. Vorzugsweise ist ein Verformungswiderstand gegen eine elastische Verformung eines an den zweiten Grenzbereich angrenzenden Halterungsmaterialbereichs des Halterungselements größer als ein Verformungswiderstand gegen eine elastische Verformung eines an den zweiten Grenzbereich angrenzenden Verbindungsmaterialbereichs des Verbindungselements. Vorzugsweise ist die elastische Verformung des Halterungsmaterialbereichs und die elastische Verformung des Verbindungsmaterialbereichs jeweils eine Biegeverformung. Vorzugsweise besteht der Halterungsmaterialbereich aus dem Halterungsmaterial und besteht der Verbindungsmaterialbereichs aus dem Halterungsmaterial verschiedenen Verbindungsmaterial. Vorzugsweise ist ein Elastizitätsmodul des Halterungsmaterials größer ist als ein Elastizitätsmodul des Verbindungsmaterials. Vorzugsweise ist das Halterungsmaterial ein Hartmetall (*cemented carbide*), ein Cermet (*cermet*), ein keramisches Material, ein Refraktärmetall oder eine Mischung daraus. Vorzugsweise ist der an den zweiten Grenzbereich angrenzende Halterungsmaterialbereich der an den (ersten) Grenzbereich angrenzende Halterungsmaterialbereich. Vorzugsweise ist das Verbindungselement monolithisch.

Gemäß einer Weiterbildung des Schneidwerkzeugs ist das Verbindungsmaterial ein metallisches Material, insbesondere ein Stahl. Hierbei handelt es sich mit Vorteil für die Herstellung einer Ausnehmung zum formschlüssigen Verbinden mit dem Antriebselement in dem Verbindungselement um ein besonders einfach zu bearbeitendes sowie relativ kostengünstiges Material.

Gemäß einer Weiterbildung des Schneidwerkzeugs sind das Halterungselement und das Verbindungselement mittels einer Klebeverbindung, einer Lötverbindung oder einer Schweißverbindung, insbesondere einer Strahlschweißverbindung, stoffschlüssig miteinander verbunden. Dadurch wird jeweils eine besonders vorteilhafte stoffschlüssige Verbindung zwischen dem Halterungselement und dem Verbindungselement bereitgestellt. Mit der Strahlschweißverbindung ist insbesondere gemeint, dass eine zum Aufschmelzen des Halterungsmaterials und/oder des Verbindungsmaterials erforderliche Schmelzenergie aus einem oder mehreren Laserstrahlen und/oder Elektronenstrahlen erhältlich ist.

Gemäß einer Weiterbildung des Schneidwerkzeugs sind in einem Längsschnitt des Schneidwerkzeugs eine erste Oberfläche und eine an die erste Oberfläche angrenzende zweite Oberfläche einen kleiner 180° bemessenen Innenkantenwinkel bildend angeordnet. Dadurch kann in vorteilhafterweise das Kollisionsrisiko einer mit dem Schneidwerkzeug verbundenen Antriebseinheit mit dem nichtmetallischen Material verringert werden, insbesondere zur Realisierung einer geradlinigen Führung des Schneidwerkzeugs, wenn das Schneidelement, insbesondere nahezu, flächenmäßig ein anderes Bauteil kontaktiert. Dies kann auch als Kröpfung des Schneidwerkzeugs bezeichnet werden. Vorzugsweise ist der Innenkantenwinkel, welcher auch als erster Innenkantenwinkel bezeichnet werden kann, kleiner oder gleich 180°, 175°, 170°, 165°, 160°, 155°, 150°, 145°, 140°, 135°, 130°, 125°, 120°, 115°, 110°, 105°, 100°, 95° oder 90°. Vorzugsweise ist der Innenkantenwinkel größer 90° und kleiner 180°, einschließlich 175°, 170°, 165°, 160°, 155°, 150°, 145°, 140°, 135°, 130°, 125°, 120°, 115°, 110°, 105°, 100°, 95°. Vorzugsweise wird danach ein Stich- und/oder Tauchsägeblatt in Form des Schneidwerkzeugs optional bereitgestellt. Vorzugsweise sind die erste und die zweite Oberfläche Oberflächen des optionalen Verbindungselements und/oder des Halterungselements. Vorzugsweise ist eine der Oberflächen eine Oberfläche des Halterungselements und die andere eine Oberfläche des Verbindungselements. Vorzugsweise ist eine der Oberflächen eine Oberfläche des Halterungselements und die andere eine Oberfläche des Schneidelements.

Gemäß einer Weiterbildung des Schneidwerkzeugs ist eine axiale Abmessung des Grenzbereichs und/oder des zweiten Grenzbereichs kleiner als eine axiale Abmessung des Halterungselements. Dadurch kann in vorteilhafterweise eine Lokalisierung der elastischen Verformung, zum Beispiel in Form eines Maximums einer Biegelinie, außerhalb des Grenzbereichs bzw. des zweiten Grenzbereichs bereitgestellt werden. Mit axialer Abmessung ist insbesondere eine parallel zu einer Längsachse des Schneidwerkzeugs bemessene Abmessung gemeint.

Gemäß einer Weiterbildung des Schneidwerkzeugs ist eine axiale Abmessung des Halterungselements größer oder gleich einer axialen Abmessung des Schneidelements. Dadurch wird die Biegesteifigkeit des Schneidwerkzeugs in vorteilhafterweise noch mehr gesteigert. Mit axialer Abmessung ist insbesondere eine parallel zu einer Längsachse des Schneidwerkzeugs bemessene Abmessung gemeint.

Gemäß einer Weiterbildung des Schneidwerkzeugs ist bzw. sind der Grenzbereich und/oder zweite Grenzbereich linienförmig, insbesondere in Draufsicht auf das Schneidwerkzeug. Danach handelt es sich bei dem Grenzbereich bzw. dem zweiten Grenzbereich zum Beispiel um eine Schweißnaht oder eine Lötnaht oder eine Klebenaht.

Gemäß einer Weiterbildung des Schneidwerkzeugs weist das Halterungselement eine Verjüngung, vorzugsweise quer zu einer Längsachse des Schneidwerkzeugs, auf. Hierbei handelt es sich um eine besonders vorteilhafte Form des Halterungselements. Vorzugsweise ist die Verjüngung am Grenzbereich und/oder zweiten Grenzbereich ausgebildet.

Das Schneidelement weist Schneidzähne auf. Dadurch wird die Trennleistung des Schneidwerkzeugs in vorteilhafterweise erhöht. Vorzugsweise bilden die Schneidzähne eine Schneidzahnreihe aus beispielsweise mindestens 10 oder mehr, beispielsweise mehr als 20, 30, 40, 50, 60, 70, 80, 90, 100, 110, 120, 130, 140, 150, 160, 170, 180, 190, 200, 250, 300, 350, 400, 500, 600, 700, 800, 900 oder 1000 Schneidzähne, nach außen spitz zulaufenden dreieckförmigen Zähnen. Vorzugsweise ist der Schneidzahnreihe eine Begrenzungslinie zuordenbar, welche vorzugsweise gerade oder rund sein kann. Vorzugsweise sind die Schneidzähne zueinander verschränkt angeordnet.

Gemäß einer Weiterbildung des Schneidwerkzeugs ist das Schneidwerkzeug manuell führbar ausgestaltet. Dadurch kann das Schneidwerkzeug insbesondere mittels eines relativ geringen Kraftaufwandes entlang einer Schnittlinie geführt werden. Vorzugsweise kann das Schneidwerkzeug nach dieser Weiterbildung mit einer handführbaren Werkzeugmaschine und/oder einem Elektrowerkzeug handführbaren verbunden werden.

Gemäß einer Weiterbildung ist das Schneidwerkzeug vorzugsweise plattenförmig ausgebildet. Hierbei handelt es sich um eine besonders einfach herzustellende Form.

Gemäß einer Weiterbildung des Schneidwerkzeugs ist diesem in einer Seitenansicht eine Dickenabmessung zuordenbar, wobei eine entsprechende Dicke entlang einer Längsachse des Schneidwerkzeugs vorzugsweise veränderlich ist, insbesondere im Bereich des Halterungselements und/oder des Schneidelements und/oder des Verbindungselements. Vorzugsweise ist die Dicke im Bereich des Verbindungselements größer als im Bereich des Schneidelements. Dies trägt zur weiteren Stabilisierung des Schneidwerkzeugs bei. Alternativ kann die Dicke jeweils im Bereich des Halterungselements und des Schneidelements und optional im Bereich des Verbindungselements kontant sein, wobei vorzugsweise die Dicke im Bereich des Halterungselements von der Dicke im Bereich des Schneidelements und/oder des Verbindungselements verschieden sein kann. Wenn die Dicke in diesen Bereichen betragsmäßig dieselbe ist, kann das Schneidwerkzeug besonders kostengünstig hergestellt werden, insbesondere mittels Schleifen oder dergleichen.

Gemäß einer Weiterbildung des Schneidwerkzeugs ist diesem in einer Draufsicht eine Breitenabmessung, sprich Breite, zuordenbar, wobei eine entsprechende Breite entlang einer Längsachse des Schneidwerkzeugs vorzugsweise veränderlich ist, insbesondere im Bereich des Halterungselements und/oder des Schneidelements und/oder des Verbindungselements. Vorzugsweise ist die Breite im Bereich des Verbindungselements größer als im Bereich des Schneidelements. Dies trägt zur weiteren Stabilisierung des Schneidwerkzeugs bei. Alternativ kann die Breite jeweils im Bereich des Halterungselements und des Schneidelements und optional im Bereich des Verbindungselements kontant sein, wobei vorzugsweise die Breite im Bereich des Halterungselements von der Breite im Bereich des Schneidelements und/oder des Verbindungselements verschieden sein kann.

Gemäß einer Weiterbildung des Schneidwerkzeugs ist diesem in einer Draufsicht eine Breitenabmessung, sprich eine Breite, und in einer Seitenansicht eine Dickenabmessung, sprich Dicke, zuordenbar, wobei eine entsprechende Breite und eine entsprechende Dicke entlang einer Längsachse des Schneidwerkzeugs vorzugsweise veränderlich sind, insbesondere im Bereich des Halterungselements und/oder des Schneidelements und/oder des Verbindungselements. Vorzugsweise ist die Breite und/oder die Dicke im Bereich des Verbindungselements größer als im Bereich des Schneidelements. Dies trägt zur weiteren Stabilisierung des Schneidwerkzeugs bei. Alternativ kann die Breite und/oder Dicke jeweils im Bereich des Halterungselements und des Schneidelements und optional im Bereich des Verbindungselements kontant sein, wobei vorzugsweise die Dicke und/oder die Breite im Bereich des Halterungselements von der Dicke und/oder Breite im Bereich des Schneidelements und/oder des Verbindungselements verschieden sein kann.

Weitere Vorteile und Zweckmäßigkeiten der Erfindung ergeben sich anhand der nachfolgenden Beschreibung von Ausführungsbeispielen unter Bezugnahme auf die beigefügten Figuren.

Von den Figuren zeigen:
- Fig. 1:: eine schematische Darstellung eines Schneidwerkzeugs nach einer ersten Ausführungsform in Draufsicht;
- Fig. 2:: eine schematische Darstellung des Schneidwerkzeugs nach Fig. 1 in einer Seitenansicht;
- Fig. 3:: eine schematische Darstellung eines Schneidwerkzeugs nach einer zweiten Ausführungsform in Draufsicht;
- Fig. 4:: eine schematische Darstellung eines Schneidwerkzeugs nach einer dritten Ausführungsform in Draufsicht;
- Fig. 5:: eine schematische Darstellung des Schneidwerkzeugs nach Fig. 4 in einer Seitenansicht;
- Fig. 6:: eine schematische Darstellung eines Schneidwerkzeugs nach einer vierten Ausführungsform in Draufsicht;
- Fig. 7:: eine schematische Darstellung des Schneidwerkzeugs nach Fig. 6 in einer Seitenansicht;
- Fig. 8:: eine schematische Darstellung eines Schneidwerkzeugs nach einer fünften Ausführungsform in Draufsicht;
- Fig. 9:: eine schematische Darstellung des Schneidwerkzeugs nach Fig. 8 in einer Seitenansicht;
- Fig. 10:: eine schematische Darstellung eines Schneidwerkzeugs nach einer sechsten Ausführungsform in Draufsicht; und
- Fig. 11:: eine schematische Darstellung des Schneidwerkzeugs nach Fig. 10 in einer Seitenansicht.

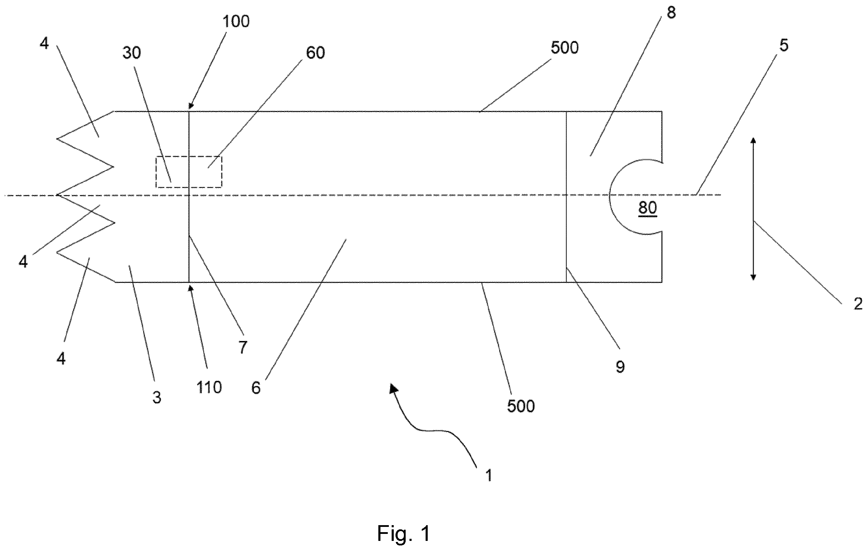

In den Figuren 1 bis 11 werden gleiche, ähnliche oder gleichwirkende Elemente mit identischen Bezugszeichen bezeichnet und auf eine wiederholte Beschreibung dieser Elemente wird in der nachfolgenden Beschreibung verzichtet, um Redundanzen zu vermeiden.

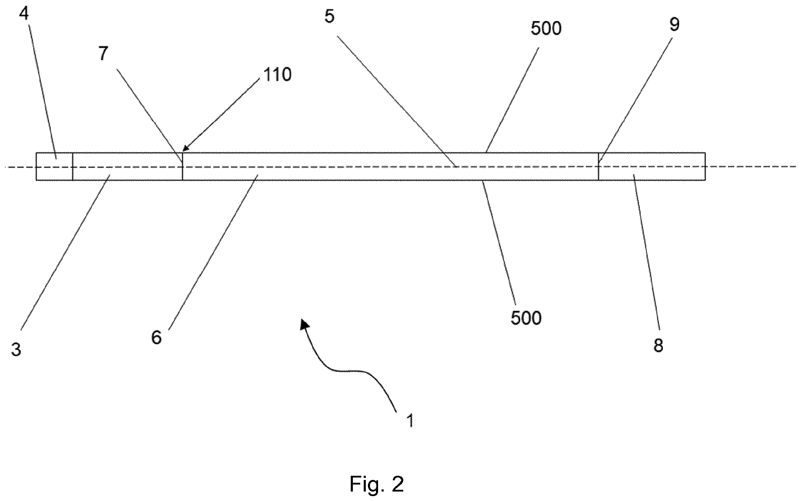

Fig. 1 und 2 zeigen eine schematische Darstellung eines plattenförmigen Schneidwerkzeugs 1 nach einer ersten Ausführungsform in Draufsicht beziehungsweise in einer Seitenansicht. Das Schneidwerkzeug 1 ist in Form eines Oszillationssägeblattes realisiert, welches entlang der Richtungen 2 mit einer Frequenz von beispielsweise 20000 Hüben pro Minute ozillizierend bewegt werden kann. Bei einer solchen Oszillation wird eine plattenförmige Schneidplatte 3, welche aus einem Stahl besteht, in Kontakt mit einem nichtmetallischen Material, zum Beispiel einem Holz oder einem Knochenmaterial, gebracht. Dabei erfolgt mittels dreieckförmiger Schneidzähne 4, deren in Fig. 1 ersichtliche Anzahl lediglich exemplarisch gewählt wurde, der Schneidplatte 3 eine Zerspanung des nichtmetallischen Materials. Eine entsprechende Schnitttiefe bzw. ein Vorschub kann dabei über eine axiale Bewegung parallel zu einer Längsachse 5 des Schneidwerkzeugs 1 in Richtung der Schneidzähne 4 eingestellt werden. Die Schneidplatte 3 ist mit einer Halterungsplatte 6 stoffschlüssig über eine Schweißnaht 7 verbunden. Die Schweißnaht 7, deren Erstreckung in axialer Richtung wesentlich kleiner ist als die der Halterungsplatte 6 und der Schneidplatte 3, bildet also einen Grenzbereich, welcher auch als Verbindungsbereich bezeichnet werden kann, zwischen der Schneidplatte 3 und der Halterungsplatte 6. Dadurch kann die Halterungsplatte 6 die bei der Zerspanung auftretenden Kräfte, welche auch als Schnittreaktionskräfte bezeichnet werden können, aufnehmen. Die Halterungsplatte 6 ist monolithisch und besteht aus einem Hartmetall (*cemented carbide*) mit einem Elastizitätsmodul von 650·10⁹ N/m²; denkbar und auch möglich sind aber auch andere Materialien im Sinne der vorliegenden Offenbarung. Die Schneidplatte 3 ist ebenfalls monolithisch, wobei sie aus einem Stahl mit einem Elastizitätsmodul von 210·10⁹ N/m² besteht. Damit ist der an die Schweißnaht 7 angrenzende Bereich 60 der Halterungsplatte 6 biegesteifer als der an die Schweißnaht 7 angrenzende Bereich 30 der Schneidplatte 3. Dies führt dazu, dass die Halterungsplatte 7 einer Auslenkung des Schneidelements 4 in eine der Richtungen senkrecht zur Längsachse 5, zum Beispiel in die Richtungen 2 oder eine der Richtungen, welche senkrecht zur Zeichnungsebene der Fig. 1 orientiert sind, und parallel dazu entgegenwirkt, so dass der Einfluss von Schnittreaktionskräften, welche auf die Schneidplatte 3 im Bereich der Schneidzähne 4 einwirken können, auf die Position der Schneidplatte 3 bezüglich eines außerhalb des Schneidwerkzeugs 1 befindlichen sowie ortsfesten Bezugspunkts reduziert wird. Der Bezugspunkt kann also beispielsweise einer Spannvorrichtung zum Spannen sowie zum Haltern des nichtmetallischen Materials zugeordnet werden. Indem die Schneidplatte 3 aus einem Stahl besteht, können die Schneidzähne 4 durch Stanzen mit anschließendem Schleifen hergestellt werden.

In Fig. 1 ist besonders gut ersichtlich, dass sich die Schweißnaht 7 über die gesamte Breite der Halterungsplatte 7 und der Schneidplatte 3 erstreckt, linienförmig ist und senkrecht zur Längsachse orientiert ist (denkbar und auch möglich ist auch eine andere Orientierung, zum Beispiel im Falle einer anderen Schneidplatte 3 und/oder einer anderen Halterungsplatte 6 parallel zur Längsachse 5 oder gegenüber der Längsachse 5 geneigt, sprich im letzteren Fall schräg verlaufend). Die Schweißnaht 7 kann daher im geometrischen Sinne als Verbindungslinie zwischen zwei Punkten 100 und 110 entsprechender Außenkanten 500 des Schneidwerkzeugs 1 betrachtet werden.

Die Halterungsplatte 6 ist ferner mit einer Verbindungsplatte 8 über eine andere Schweißnaht 9 verbunden. Die Verbindungsplatte 8 ist ebenfalls monolithisch, wobei sie aus einem Stahl mit einem Elastizitätsmodul von 210·10⁹ N/m² besteht. Indem die Verbindungsplatte 8 aus Stahl besteht, ist eine konkave Ausnehmung 80 der Verbindungsplatte 8 durch Stanzen erhältlich, wobei die Ausnehmung 80 einer nicht dargestellten Antriebswelle eines Elektromotors korrespondierend ausgeformt ist, so dass das Schneidwerkzeug 1 mittels des Elektromotors zur Ausführung der Oszillationsbewegungen in die Richtungen 2 angetrieben werden kann. Auf diese Weise wird ein Elektrowerkzeug bereitgestellt.

In Fig. 2 ist besonders gut ersichtlich, dass das Schneidwerkzeug 1 eine konstante Dicke, welche auch als Plattenstärke bezeichnet werden kann, aufweist, welche folglich im Bereich der Schneidplatte 3, der Halterungsplatte 6 und der Verbindungsplatte 8 betragsmäßig dieselbe ist.

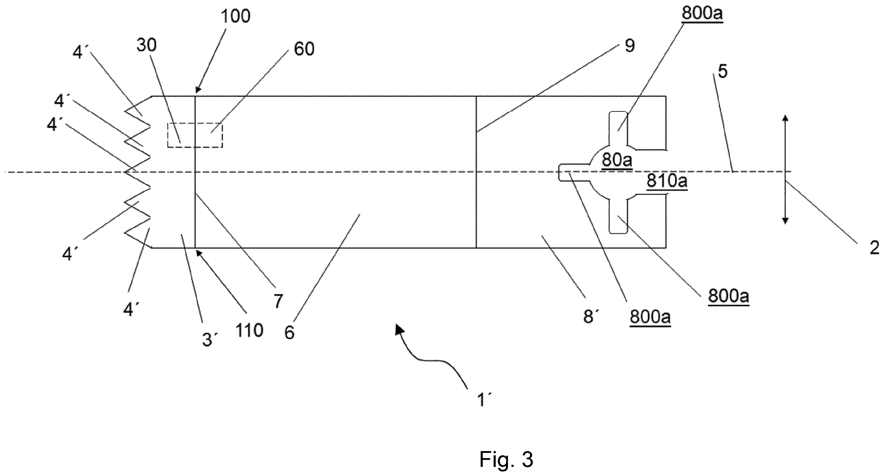

Fig. 3 zeigt eine schematische Darstellung eines plattenförmigen Schneidwerkzeugs 1' nach einer zweitem Ausführungsform in Draufsicht beziehungsweise in einer Seitenansicht. Das Schneidwerkzeug 1' ist dem Schneidwerkzeug 1 analog aufgebaut, wobei im Unterschied zum Schneidwerkzeug 1 eine Verbindungsplatte 8', welche aus dem Stahl der Verbindungsplatte 8 besteht, eine Ausnehmung 80a mit radialen Auskragungen 800a und einer axialen Stecknut 810a aufweist, welche einer nicht dargestellten Antriebswelle der Elektromotors korrespondierend ausgeformt sind, um gegenüber der Ausnehmung 80 mehr Formschlusskräfte auf das Schneidwerkzeug 1' zu übertragen. Ferner ist die Verbindungsplatte 8' in axialer Richtung länger als die Verbindungsplatte 8 der Schneidwerkzeugs 1, was den flächenmäßigen Kontakt mit einer Einspannvorrichtung einer Elektrowerkzeugantriebseinheit erhöht. Ferner ist die Schneidplatte 3' in axialer Richtung kürzer als die Schneidplatte 3, was die Schneidplatte 4'biegesteifer macht. Die Anzahl der Schneidzähne 4' ist exemplarisch größer als die Anzahl der Schneidzähne 4.

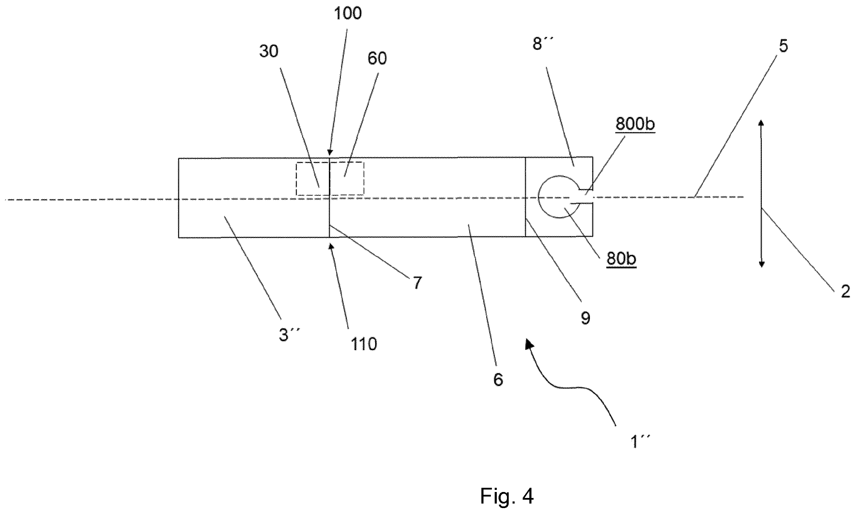

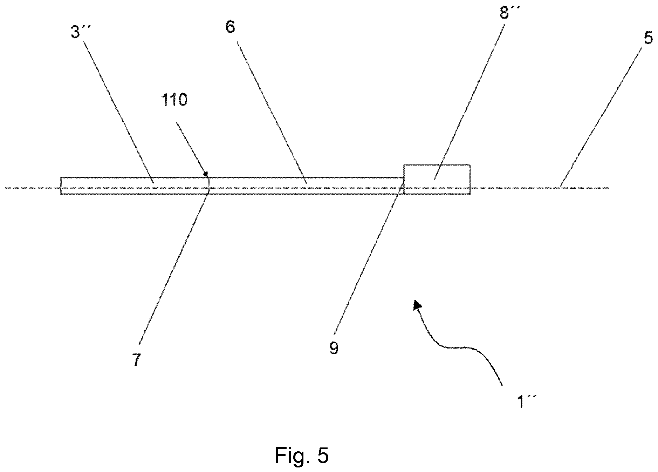

Fig. 4 und 5 zeigen eine schematische Darstellung eines plattenförmigen Schneidwerkzeugs 1" nach einer dritten Ausführungsform in Draufsicht beziehungsweise in einer Seitenansicht. Das Schneidwerkzeug 1" ist dem Schneidwerkzeug 1 analog aufgebaut, wobei das Schneidelement 3", welches aus dem Stahl des Schneidelements 3 besteht, ohne Schneidzähne dargestellt ist und eine Verbindungsplatte 8", welche aus dem Stahl der Verbindungsplatte 8 besteht, eine Ausnehmung 80b mit einer Stecknut 800 b aufweist.

In Fig. 5 ist besonders gut ersichtlich, dass das Schneidwerkzeug 1" eine konstante Dicke, welche auch als Plattenstärke bezeichnet werden kann, im Bereich der Schneidplatte 3" und der Halterungsplatte 6 aufweist, welche in diesen Bereichen betragsmäßig dieselbe ist, und eine konstante Dicke, also Plattenstärke, im Bereich der Verbindungsplatte 8" aufweist, welche größer ist als die Plattenstärke in den Bereichen der Schneidplatte 3" und der Halterungsplatte 6.

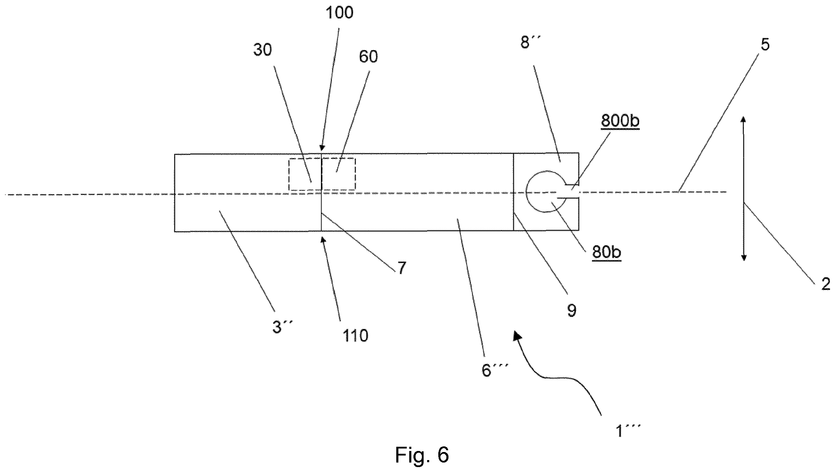

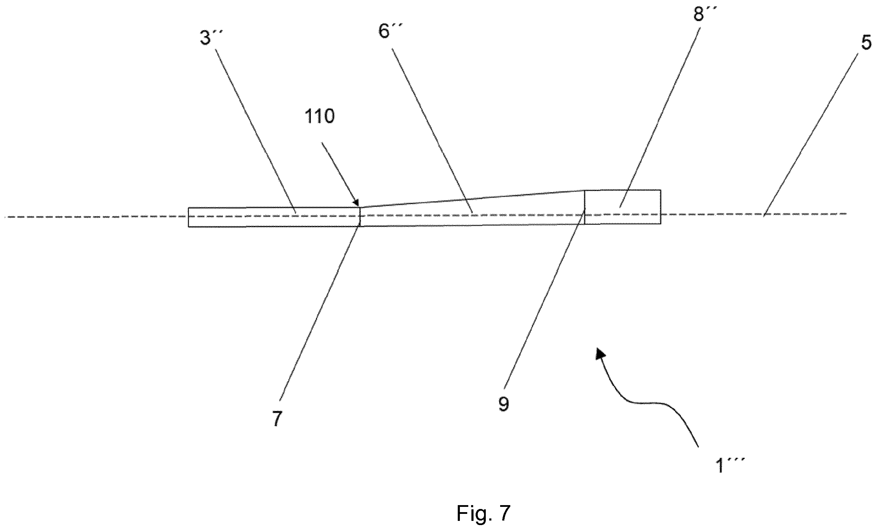

Fig. 6 und 7 zeigen eine schematische Darstellung eines plattenförmigen Schneidwerkzeugs 1‴ nach einer vierten Ausführungsform in Draufsicht beziehungsweise in einer Seitenansicht. Das Schneidwerkzeug 1‴ ist dem Schneidwerkzeug 1" analog aufgebaut, wobei, wie aus Fig. 7 besonders gut ersichtlich ist, ein Dicke, also Plattenstärke, des Schneidwerkzeugs 1‴ im Bereich einer Halterungsplatte 6‴, beginnend an der Schweißnaht 7 bei einer Dicke, welche der Dicke der Schneidplatte 3" entspricht, kontinuierlich größer wird, bis die Dicke der Verbindungsplatte 8" an der Schweißnaht 9 erreicht ist. Die Halterungsplatte 6‴ besteht dabei aus dem Hartmetall *(cemented carbide)* der Halterungsplatte 6.

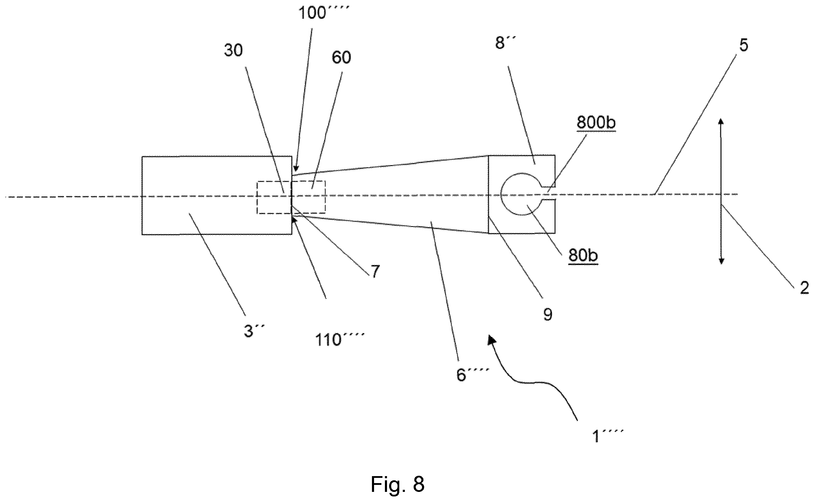

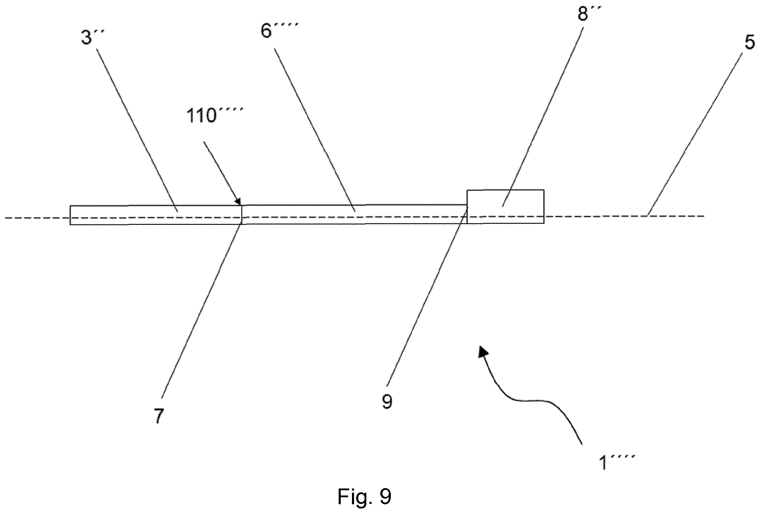

Fig. 8 und 9 zeigen eine schematische Darstellung eines plattenförmigen Schneidwerkzeugs 1ʺʺ nach einer fünften Ausführungsform in Draufsicht beziehungsweise in einer Seitenansicht. Das Schneidwerkzeug 1ʺʺ ist dem Schneidwerkzeug 1‴ analog aufgebaut, wobei eine Halterungsplatte 6ʺʺ, wie aus Fig. 8 besonders gut ersichtlich ist, eine quer zur Längsachse 2 bemessene Breite aufweist, welche von der Schweißnaht 9 nach der Schweißnaht 7 kontinuierlich kleiner wird; an der Schweißnaht 9 entspricht die Breite einer analog bemessenen Breite der Verbindungsplatte 8". Danach weist das Schneidwerkzeug 1ʺʺ eine Verjüngung auf, und zwar im Bereich der Halterungsplatte 6ʺʺ. Die Breite der Halterungsplatte 6ʺʺ entspricht an der Schweißnaht 7 dem Abstand zwischen Punkten 100ʺʺ und 110ʺʺ entsprechender Außenkanten des Schneidwerkzeugs 1ʺʺ. Die Halterungsplatte 6ʺʺ besteht dabei aus dem Hartmetall *(cemented carbide)* der Halterungsplatte 6.

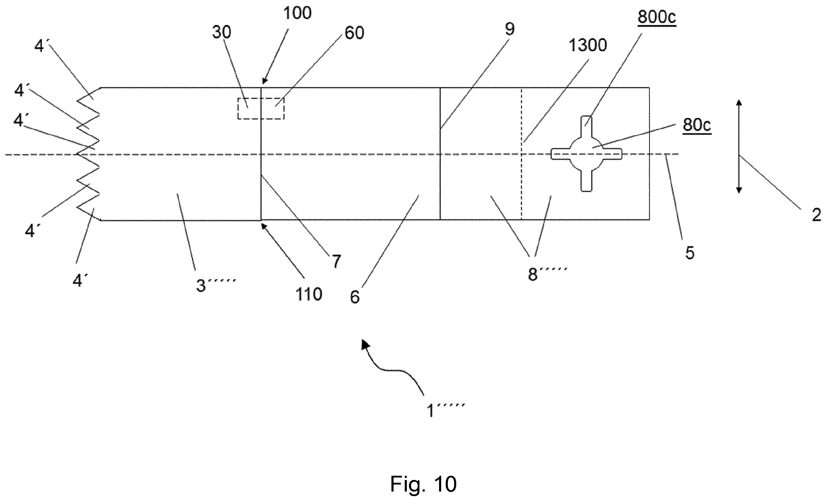

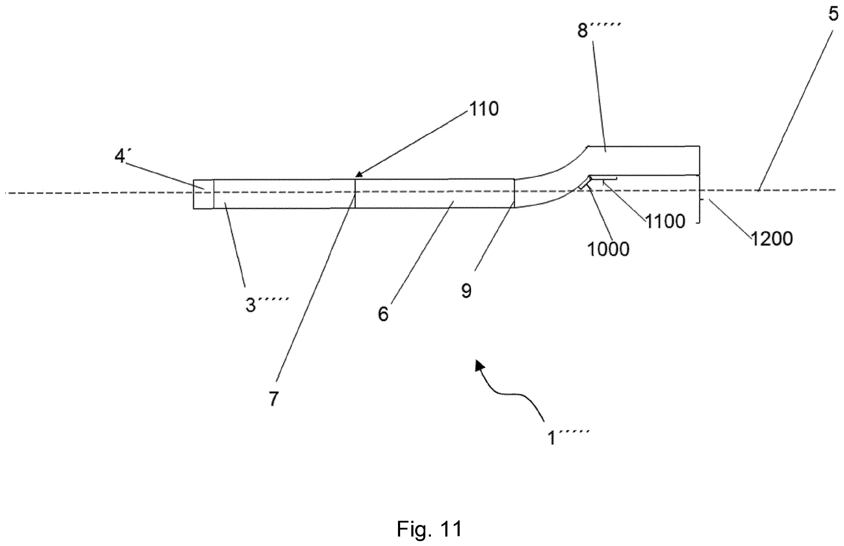

Fig. 10 und 11 zeigen eine schematische Darstellung eines plattenförmigen Schneidwerkzeugs 1‴ʺ nach einer sechsten Ausführungsform in Draufsicht beziehungsweise in einer Seitenansicht. Das Schneidwerkzeug 1‴ʺ ist dem Schneidwerkzeug 1' analog aufgebaut, wobei, wie aus Fig. 11 besonders gut ersichtlich ist, eine Verbindungsplatte 8‴ʺ, welche aus dem Stahl der Verbindungsplatte 8 besteht, zwei aneinander angrenzende Oberflächen 1000 und 1100, welche in der Seitenansicht gemäß Fig. 11 analog zu einem entsprechenden Längsschnitt im Profil ersichtlich sind, einen mit 135° bemessenen Innenkantenwinkel bilden. Auf diese Weise ist das Schneidwerkzeug 1‴ʺ im Bereich der Verbindungsplatte 8‴ʺ derart gekröpft, dass gegenüber zum Beispiel dem Schneidwerkzeug 1 ein in Fig. 11 ersichtlicher Abstand 1200 quer zu Längsachse 5 im Bereich der Verbindungsplatte 8‴ʺgewonnen wird, so dass der Abstand 1200 im Sinne eines zusätzlichen Montageraums zum Verbinden mit einem Elektromotor einer Antriebseinheit oder dergleichen verwendet werden kann, wobei - bei Einhaltung der Grenzen des Abstands 1200 - eine Schneidplatte 3‴ʺ zugleich einen Untergrund oder dergleichen parallel zu ihrer in Fig. 10 ersichtlichen flächenmäßigen Erstreckung kontaktieren kann. Ein Übergang von der Oberfläche 1000 nach der Oberfläche 1100 beziehungsweise umgekehrt, ist mit einer gestrichelten Line 1300, welche parallel zu einer Querachse verläuft, in Fig. 10 markiert. Die Verbindungsplatte 8‴ʺ weist eine radial geschlossene Ausnehmung 80c mit Auskragungen 800c zum formschlüssigen Verbinden mit einer Welle eines Elektromotors auf.

Die Schneidplatte 3‴ʺ, welche aus dem Stahl der Schneidplatte 3 besteht, ist länger als die Schneidplatte 3', um axialen Bearbeitungsraum zum Trennen des nichtmetallischen Materials zu gewinnen.

## Patentansprüche

1. Schneidwerkzeug (1, 1', 1", 1‴, 1ʺʺ, 1‴ʺ) zum Trennen eines nichtmetallischen Materials, umfassend ein Halterungselement (6, 6", 6‴, 6ʺʺ), ein an dem Halterungselement (6, 6", 6‴, 6ʺʺ) zur schneidenden Kontaktierung des nichtmetallischen Materials gehaltertes Schneidelement (3, 3', 3", 3‴ʺ) und einen an einen Halterungsmaterialbereich (60) des Halterungselements (6, 6", 6‴, 6ʺʺ) sowie einen Schneidmaterialbereich (30) des Schneidelements (3, 3', 3", 3‴ʺ) angrenzenden Grenzbereich (7), wobei ein Verformungswiderstand gegen eine elastische Verformung des Halterungsmaterialbereichs (60) größer ist als ein Verformungswiderstand gegen eine elastische Verformung des Schneidmaterialbereichs (30), wobei das Halterungselement (6, 6", 6‴, 6ʺʺ) und das Schneidelement (3, 3', 3'', 3‴ʺ) stoffschlüssig miteinander verbunden sind, wobei das Schneidelement (3, 3', 3", 3‴ʺ Schneidzähne (4) aufweist, **dadurch gekennzeichnet, dass** der Halterungsmaterialbereich (60) aus einem Halterungsmaterial besteht und der Schneidmaterialbereich (30) aus einem dem Halterungsmaterial verschiedenen Schneidmaterial besteht, wobei ein Elastizitätsmodul des Halterungsmaterials größer ist als ein Elastizitätsmodul des Schneidmaterials.

2. Schneidwerkzeug (1, 1', 1", 1‴, 1ʺʺ, 1‴ʺ) nach Anspruch 1, wobei der Verformungswiderstand des Halterungsmaterialbereichs (60) und der Verformungswiderstand des Schneidmaterialbereichs (30) jeweils ein Biegeverformungswiderstand ist.

3. Schneidwerkzeug (1, 1', 1", 1‴, 1ʺʺ, 1‴ʺ) nach einem der vorhergehenden Ansprüche, wobei das Halterungsmaterial eine Materialmatrix mit einer Matrixduktilität und in der Materialmatrix angeordnete Hartstoffpartikel mit einer Partikelduktilität, welche kleiner ist als die Matrixduktilität, aufweist.

4. Schneidwerkzeug (1, 1', 1", 1‴, 1ʺʺ, 1‴ʺ) nach einem der vorhergehenden Ansprüche, wobei das Halterungsmaterial ein keramisches Material, ein Refraktärmetall oder eine Mischung daraus ist.

5. Schneidwerkzeug (1, 1', 1", 1‴, 1ʺʺ, 1‴ʺ) nach einem der vorhergehenden Ansprüche, wobei das Halterungselement (6, 6", 6‴, 6ʺʺ) monolithisch ist.

6. Schneidwerkzeug (1, 1', 1", 1‴, 1ʺʺ, 1‴ʺ) nach einem der vorhergehenden Ansprüche, wobei das Schneidmaterial ein metallisches Material, insbesondere ein Stahl, ist und eine Duktilität des Schneidmaterials größer ist als eine Duktilität des Halterungsmaterials.

7. Schneidwerkzeug (1, 1', 1", 1‴, 1ʺʺ, 1‴ʺ) nach einem der vorhergehenden Ansprüche, wobei der Halterungsmaterialbereich (60) und der Schneidmaterialbereich (30) mittels einer Klebeverbindung, einer Lötverbindung oder einer Schweißverbindung (7), insbesondere einer Strahlschweißverbindung, stoffschlüssig miteinander verbunden sind.

8. Schneidwerkzeug (1, 1', 1", 1‴, 1ʺʺ, 1‴ʺ) nach einem der vorhergehenden Ansprüche, wobei der Grenzbereich (7) zwischen zwei Außenkantenpunkten (100, 110, 100ʺʺ, 110ʺʺ) des Schneidwerkzeugs (1, 1', 1", 1‴, 1ʺʺ, 1‴ʺ) erstreckend ausgeformt ist.

9. Schneidwerkzeug (1, 1', 1", 1‴, 1ʺʺ, 1‴ʺ) nach einem der vorhergehenden Ansprüche, wobei ein Außenbereich des Halterungsmaterialbereichs (60) zumindest abschnittsweise eine Fase oder eine Kantenverrundung aufweist.

10. Schneidwerkzeug (1, 1', 1", 1‴, 1ʺʺ, 1‴ʺ) nach einem der vorhergehenden Ansprüche, wobei zwei Außenkanten (500) des Halterungsmaterialbereich (60) durch eine konvexe Sehne verbunden sind.

11. Schneidwerkzeug (1, 1', 1", 1‴, 1ʺʺ, 1‴ʺ) nach einem der vorhergehenden Ansprüche, wobei das Schneidwerkzeug (1, 1', 1", 1‴, 1ʺʺ, 1‴ʺ) ein Verbindungselement (8, 8', 8", 8‴ʺ) zum formschlüssigen Verbinden mit einem Antriebselement einer Antriebeinheit umfasst, wobei das Verbindungselement (8, 8', 8", 8‴ʺ) an dem Halterungselement (6, 6", 6‴, 6ʺʺ) gehaltert ist, ein Grenzbereich (9) zwischen dem Verbindungselement (8, 8', 8", 8‴ʺ) und dem Halterungselement (6, 6", 6‴, 6ʺʺ) gebildet ist und eine Duktilität eines Verbindungsmaterials des Verbindungselements (8, 8', 8", 8‴ʺ) größer ist als eine Duktilität des Halterungsmaterials.

12. Schneidwerkzeug (1, 1', 1", 1‴, 1ʺʺ, 1‴ʺ) nach Anspruch 11, wobei das Verbindungsmaterial ein metallisches Material, insbesondere ein Stahl, ist.

13. Schneidwerkzeug (1, 1', 1", 1‴, 1ʺʺ, 1‴ʺ) nach Anspruch 11 oder 12, wobei das Halterungselement (6, 6", 6‴, 6ʺʺ) und das Verbindungselement (8, 8', 8", 8‴ʺ) mittels einer Klebeverbindung, einer Lötverbindung oder einer Schweißverbindung (9), insbesondere einer Strahlschweißverbindung, stoffschlüssig miteinander verbunden sind.

14. Schneidwerkzeug (1, 1', 1", 1‴, 1ʺʺ, 1‴ʺ) nach einem der vorhergehenden Ansprüche, wobei in einem Längsschnitt des Schneidwerkzeugs (1, 1', 1", 1‴, 1ʺʺ, 1‴ʺ) eine erste Oberfläche (1000) und eine an die erste Oberfläche (1000) angrenzende zweite Oberfläche (1100) einen kleiner 180° bemessenen Innenkantenwinkel bildend angeordnet sind.

## Claims

1. Cutting tool (1, 1', 1'', 1‴, 1ʺʺ, 1‴ʺ) for severing a non-metallic material, comprising a holding element (6, 6", 6‴, 6ʺʺ)**,** a cutting element (3, 3', 3'', 3‴ʺ), which is held on the holding element (6, 6'', 6‴**,** 6ʺʺ) for the purpose of making cutting contact with the non-metallic material, and a boundary region (7), which adjoins a holding material region (60) of the holding element (6, 6'', 6‴**,** 6ʺʺ) and a cutting material region (30) of the cutting element (3, 3', 3'', 3‴ʺ), wherein a deformation resistance to elastic deformation of the holding material region (60) is greater than a deformation resistance to elastic deformation of the cutting material region (30), wherein the holding element (6, 6", 6‴, 6ʺʺ) and the cutting element (3, 3', 3"**,** 3‴ʺ) are connected to one another in a materially bonded manner, wherein the cutting element (3, 3', 3'', 3‴ʺ) has cutting teeth (4), **characterized in that** the holding material region (60) consists of a holding material and the cutting material region (30) consists of a cutting material, which is different from the holding material, wherein a modulus of elasticity of the holding material is greater than a modulus of elasticity of the cutting material.

2. Cutting tool (1, 1', 1'', 1‴, 1ʺʺ, 1‴ʺ) according to Claim 1, wherein each of the deformation resistance of the holding material region (60) and the deformation resistance of the cutting material region (30) is a bending deformation resistance.

3. Cutting tool (1, 1', 1'', 1‴, 1ʺʺ, 1‴ʺ) according to either of the preceding claims, wherein the holding material has a material matrix with a matrix ductility and has hard material particles, arranged in the material matrix, with a particle ductility, which is smaller than the matrix ductility.

4. Cutting tool (1, 1', 1'', 1‴, 1ʺʺ, 1‴ʺ) according to one of the preceding claims, wherein the holding material is a ceramic material, a refractory metal or a mixture thereof.

5. Cutting tool (1, 1', 1'', 1‴, 1ʺʺ, 1‴ʺ) according to one of the preceding claims, wherein the holding element (6, 6", 6‴, 6ʺʺ) is monolithic.

6. Cutting tool (1, 1', 1'', 1‴, 1ʺʺ, 1‴ʺ) according to one of the preceding claims, wherein the cutting material is a metallic material, in particular a steel, and a ductility of the cutting material is greater than a ductility of the holding material.

7. Cutting tool (1, 1', 1'', 1‴, 1ʺʺ, 1‴ʺ) according to one of the preceding claims, wherein the holding material region (60) and the cutting material region (30) are connected to one another in a materially bonded manner by means of an adhesive connection, a soldered connection or a welded connection (7), in particular a beam welded connection.

8. Cutting tool (1, 1', 1'', 1‴, 1ʺʺ, 1‴ʺ) according to one of the preceding claims, wherein the boundary region (7) is formed so as to extend between two outer edge points (100, 110, 100ʺʺ, 110ʺʺ) of the cutting tool (1, 1' , 1'', 1‴, 1ʺʺ, 1‴ʺ).

9. Cutting tool (1, 1', 1'', 1‴, 1ʺʺ, 1‴ʺ) according to one of the preceding claims, wherein an outer region of the holding material region (60) at least sectionally has a bevel or an edge rounding.

10. Cutting tool (1, 1' , 1'', 1‴, 1ʺʺ, 1‴ʺ) according to one of the preceding claims, wherein two outer edges (500) of the holding material region (60) are connected by a convex chord.

11. Cutting tool (1, 1', 1'', 1‴, 1ʺʺ, 1‴ʺ) according to one of the preceding claims, wherein the cutting tool (1, 1', 1", 1''', 1ʺʺ, 1‴ʺ) comprises a connecting element (8, 8', 8'', 8‴ʺ) for form-fitting connection to a drive element of a drive unit, wherein the connecting element (8, 8', 8", 8‴ʺ) is held on the holding element (6, 6'', 6‴, 6ʺʺ), a boundary region (9) is formed between the connecting element (8, 8', 8'', 8‴ʺ) and the holding element (6, 6", 6‴**,** 6ʺʺ) and a ductility of a connecting material of the connecting element (8, 8', 8'', 8‴ʺ) is greater than a ductility of the holding material.

12. Cutting tool (1, 1' , 1'', 1‴, 1ʺʺ, 1‴ʺ) according to Claim 11, wherein the connecting material is a metallic material, in particular a steel.

13. Cutting tool (1, 1', 1'', 1‴, 1ʺʺ, 1‴ʺ) according to Claim 11 or 12, wherein the holding element (6, 6'', 6‴, 6ʺʺ) and the connecting element (8, 8', 8'', 8‴ʺ) are connected to one another in a materially bonded manner by means of an adhesive connection, a soldered connection or a welded connection (9), in particular a beam welded connection.

14. Cutting tool (1, 1', 1'', 1‴, 1ʺʺ, 1‴ʺ) according to one of the preceding claims, wherein, in a longitudinal section of the cutting tool (1, 1', 1'', 1‴, 1ʺʺ, 1‴ʺ), a first surface (1000) and a second surface (1100), which adjoins the first surface (1000), are arranged so as to form an internal edge angle of less than 180°.

## Revendications

1. Outil de coupe (1, 1', 1", 1‴, 1ʺʺ, 1‴ʺ) pour couper un matériau non métallique, comprenant un élément de support (6, 6'', 6‴, 6ʺʺ), un élément de coupe (3, 3', 3'', 3‴ʺ) supporté sur l'élément de support (6, 6'', 6‴, 6ʺʺ) pour établir un contact de coupe avec le matériau non métallique, et une zone limite (7) adjacente à une zone de matériau de support (60) de l'élément de support (6, 6'', 6‴, 6ʺʺ) ainsi qu'à une zone de matériau de coupe (30) de l'élément de coupe (3, 3', 3"**,** 3‴ʺ), une résistance à la déformation contre une déformation élastique de la zone de matériau de support (60) étant supérieure à une résistance à la déformation contre une déformation élastique de la zone de matériau de coupe (30), l'élément de support (6, 6", 6‴**,** 6ʺʺ) et l'élément de coupe (3, 3', 3"**,** 3‴ʺ) étant reliés l'un à l'autre par liaison de matière, l'élément de coupe (3, 3', 3"**,** 3‴ʺ) présentant des dents de coupe (4), **caractérisé en ce que** la zone de matériau de support (60) est constituée d'un matériau de support et la zone de matériau de coupe (30) est constituée d'un matériau de coupe différent du matériau de support, un module d'élasticité du matériau de support étant supérieur à un module d'élasticité du matériau de coupe.

2. Outil de coupe (1, 1', 17", 1‴, 1ʺʺ, 1‴ʺ) selon la revendication 1, dans lequel la résistance à la déformation de la zone de matériau de support (60) et la résistance à la déformation de la zone de matériau de coupe (30) sont chacune une résistance à la déformation en flexion.

3. Outil de coupe (1, 1', 1", 1‴, 1ʺʺ, 1‴ʺ) selon l'une quelconque des revendications précédentes, dans lequel le matériau de support présente une matrice de matériau ayant une ductilité de matrice et des particules de matière dure agencées dans la matrice de matériau ayant une ductilité de particule qui est inférieure à la ductilité de matrice.

4. Outil de coupe (1, 1', 1", 1‴, 1ʺʺ, 1‴ʺ) selon l'une quelconque des revendications précédentes, dans lequel le matériau de support est un matériau céramique, un matériau réfractaire ou un mélange de ceux-ci.

5. Outil de coupe (1, 1', 17", 1‴, 1ʺʺ, 1‴ʺ) selon l'une quelconque des revendications précédentes, dans lequel l'élément de support (6, 6'', 6‴**,** 6ʺʺ) est monolithique.

6. Outil de coupe (1, 1', 1", 1‴, 1ʺʺ, 1‴ʺ) selon l'une quelconque des revendications précédentes, dans lequel le matériau de coupe est un matériau métallique, notamment un acier, et une ductilité du matériau de coupe est supérieure à une ductilité du matériau de support.

7. Outil de coupe (1, 1', 1", 1‴, 1ʺʺ, 1‴ʺ) selon l'une quelconque des revendications précédentes, dans lequel la zone de matériau de support (60) et la zone de matériau de coupe (30) sont reliées l'une à l'autre par liaison de matière au moyen d'une liaison par collage, d'une liaison par brasage ou d'une liaison par soudage (7), notamment d'une liaison par soudage par rayonnement.

8. Outil de coupe (1, 1', 17", 1‴, 1ʺʺ, 1‴ʺ) selon l'une quelconque des revendications précédentes, dans lequel la zone limite (7) est formée de manière à s'étendre entre deux points de bord extérieur (100, 110, 100'''', 110'''') de l'outil de coupe (1, 1', 1", 1‴, 1ʺʺ, 1‴ʺ).

9. Outil de coupe (1, 1', 1", 1‴, 1ʺʺ, 1‴ʺ) selon l'une quelconque des revendications précédentes, dans lequel une zone extérieure de la zone de matériau de support (60) présente au moins par sections un chanfrein ou un arrondi de bord.

10. Outil de coupe (1, 1', 1", 1‴, 1ʺʺ, 1‴ʺ) selon l'une quelconque des revendications précédentes, dans lequel deux bords extérieurs (500) de la zone de matériau de support (60) sont reliés par une corde convexe.

11. Outil de coupe (1, 1', 1", 1‴, 1ʺʺ**,** 1‴ʺ) selon l'une quelconque des revendications précédentes, dans lequel l'outil de coupe (1, 1', 17", 1‴, 1ʺʺ, 1‴ʺ) comprend un élément de liaison (8, 8', 8'', 8‴ʺ) destiné à être relié par complémentarité de forme à un élément d'entraînement d'une unité d'entraînement, l'élément de liaison (8, 8', 8'', 8‴ʺ) étant supporté sur l'élément de support (6, 6", 6‴, 6ʺʺ), une zone limite (9) étant formée entre l'élément de liaison (8, 8', 8", 8‴ʺ) et l'élément de support (6, 6'', 6‴**,** 6ʺʺ) et une ductilité d'un matériau de liaison de l'élément de liaison (8, 8', 8'', 8‴ʺ) étant supérieure à une ductilité du matériau de support.

12. Outil de coupe (1, 1' , 17", 1‴, 1ʺʺ, 1‴ʺ) selon la revendication 11, dans lequel le matériau de liaison est un matériau métallique, notamment un acier.

13. Outil de coupe (1, 1', 1", 1‴, 1ʺʺ, 1‴ʺ) selon la revendication 11 ou 12, dans lequel l'élément de support (6, 6", 6‴, 6ʺʺ) et l'élément de liaison (8, 8', 8"**,** 8‴ʺ) sont reliés l'un à l'autre par liaison de matière au moyen d'une liaison par collage, d'une liaison par brasage ou d'une liaison par soudage (9), notamment d'une liaison par soudage par rayonnement.

14. Outil de coupe (1, 1', 1", 1‴, 1ʺʺ, 1‴ʺ) selon l'une quelconque des revendications précédentes, dans lequel, dans une coupe longitudinale de l'outil de coupe (1, 1', 1", 1‴, 1ʺʺ, 1‴ʺ), une première surface (1000) et une deuxième surface (1100) adjacente à la première surface (1000) sont agencées de manière à former un angle de bord intérieur dimensionné à moins de 180°.
